(19) Europäisches Patentamt
European Patent Office
Office européen des brevets

(11) Publication number: **0 019 223**
**A1**

(12) **EUROPEAN PATENT APPLICATION**

(21) Application number: 80102536.2

(22) Date of filing: 08.05.80

(51) Int. Cl.³: C 07 D 233/78
A 61 K 31/415, C 07 C 127/15
C 07 C 101/20

(30) Priority: 09.05.79 GB 7916023

(43) Date of publication of application: 26.11.80 Bulletin 80/24

(84) Designated Contracting States: CH DE FR GB LI SE

(71) Applicant: THE WELLCOME FOUNDATION LIMITED
183-193 Euston Road
London NW1 2BP(GB)

(72) Inventor: Whittaker, Norman
37 Brabourne Rise
Beckenham, Kent(GB)

(74) Representative: Berg, Wilhelm, Dr. et al,
Dr. Berg, Dipl.-Ing. Stapf, Dipl.-Ing. Schwabe, Dr. Dr.
Sandmair Mauerkircherstrasse 45
D-8000 München 80(DE)

(54) **Optically active hydantoin derivatives, their synthesis, pharmaceutical formulations containing them, and intermediates.**

(57) Compounds of formula (I)

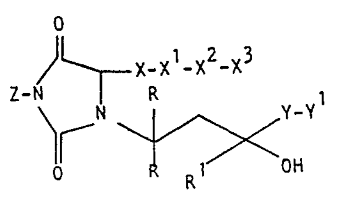

(1)

having only two optically active centres, may exist as four isomers, each of which may be prepared by resolving a racemic amino-diester intermediate in the preparation thereof. The racemic amino-diester is selectively hydrolysed to the corresponding mono-ester which is acylated and subsequently selectively deacylated to produce the resolved mono-ester.

Isomers of compounds of formula (I) having a particular configuration are biologically more active than the corresponding isomers having other configurations and are useful in medicine, either as the raw compound or in a pharmaceutical formulation, for example in inhibiting platelet aggregation.

EP 0 019 223 A1

Croydon Printing Company Ltd.

DR. BERG DIPL.-ING. STAFF
DIPL.-ING. SCHWABE DR. DR. SANDMAIR
PATENTANWÄLTE
Postfach 86 02 45 · 8000 München 86

0019223

Attorney's file: 50 087 8th May 1980

THE WELLCOME FOUNDATION LTD.
London / England

TITLE MODIFIED
see front page

Optically active heterocyclic compounds, their synthesis, and pharmaceutical formulations containing them

This invention relates to optically active heterocyclic compounds, their synthesis, separation of their optically active isomers and their use in medicine.

☎ (089) 98 82 72
98 82 73
98 82 74

Telegramme:
BERGSTAPFPATENT München
TELEX:

Bankkonten: Hypo-Bank München 4410122850
(BLZ 700 200 11) Swift Code: HYPO DE MM
Bayer. Vereinsbank München 453100 (BLZ 700 202 70)

In the complete specifications of our UK published specification No 2 032 419A, European Patent application numbers 78. 100823.0 and 78.101491.5, and Belgium Patent No 855 337 we describe certain hydantoin derivatives, including those defined hereinbelow in formula (I), which have been found to have pharmacological properties related to those of natural prostaglandins, as demonstrated by their ability to mimic or antagonise the physiological effects of the natural prostaglandins in various biological preparations. In particular, certain compounds of formula (I) have been found to be potent mimetics of the antiplatelet aggregatory properties of prostaglandin $E_1$.

In formula (I)

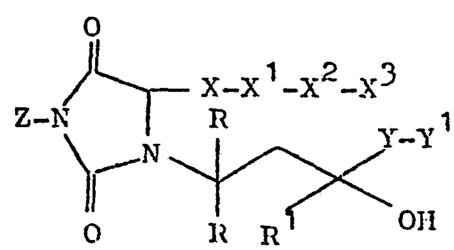

there are only two optically active centres;

BAD ORIGINAL

Z is hydrogen or alkyl of 1 to 6 carbon atoms;

X is methylene, oxa (-O-) or thia (-S-);

$X^1$ is phenylene, -C≡C-, <u>cis</u> or <u>trans</u> -CH=CH- or $-CH_2-CH_2-$;

$X^2$ is a covalent bond or a straight or branched alkylene chain having 1 to 6 carbon atoms;

$X^1$ or $X^2$ optionally has one of any methylene groups replaced by oxa or thia provided that at least one carbon atom separates the oxa or thia from any 5-tetrazolyl or carbonyl or -C≡C- or <u>cis</u> or <u>trans</u>-CH=CH- group, further provided that $-X-X^1-X^2-$ contains not more than one of either an oxa or thia;

$X^3$ is selected from 5-tetrazolyl, carboxyl, carbamoyl alkoxycarbonyl and hydroxymethyl;

each R is the same and is selected from hydrogen and methyl;

$R^1$ is hydrogen or alkyl;

Y is a covalent bond or straight or symmetrically branched alkylene having 1 to 7 carbon atoms optionally substituted at the carbon adjacent $-C{<}^{R^1}_{OH}$ by two groups each of which may be alkyl or a cyclic radical provided no optical centre is introduced;

$Y^1$ is hydrogen, alkoxy of 1 to 7, preferably 1 to 4, carbon atoms, a cyclic radical, phenyl, benzyl, phenoxy or benzyloxy, wherein each of phenyl, benzyl, phenoxy and benzyloxy may

BAD ORIGINAL

be substituted in the benzene ring by one or more groups selected from hydroxy, halo, nitro, amino, acylamino, alkenyl, alkoxy, phenyl, benzyloxy and alkyl which may itself be substituted by one or more halo groups; or Y and $Y^1$ together form an alkyl group of 1 to 7 carbon atoms having at least one hydrogen replaced by fluoro.

In the definitions of Y and $Y^1$ in formula (I), the term cyclic radical means the monovalent radical derived by loss of a ring hydrogen atom from a monocyclic or polycyclic compound having from 3 to 12 ring atoms selected from carbon, nitrogen, oxygen and sulphur, which compound may be saturated or unsaturated and may be further substituted by one or more alkyl groups, but excluding phenyl. Such cyclic radicals include cycloalkyl having 3 to 10 carbon atoms such as cyclopropyl, cyclobutyl, cyclopentyl, cyclohexyl, cycloheptyl and cyclooctyl; bicycloalkyl having 4 to 10 carbon atoms such as norbornyl (bicyclo[2,2,1]heptyl) and adamantyl, spiroalkanyl having 5 to 12 carbon atoms such as 2-spiro[3,3]heptyl, 1-spiro[4,4]nonane and 8-spiro[4,5]decane; cycloalkenyl having 4 to 10 carbon atoms such as 4-cyclopentene; heterocyclic radicals such as tetrahydrofuranyl and

BAD ORIGINAL

tetrahydropyranyl and heteroaryl radicals such as thienyl, furyl, pyridyl, pyrimidyl, thiazolyl, imidazolyl and diazepinyl. Included in the term cyclic radical are those wherein one or more hydrogen atoms are replaced by fluoro.

Unless otherwise stated, in formula (I) and other formulae in this specification, alkyl moieties are selected from methyl, ethyl, propyl, butyl, pentyl and hexyl, including all isomers thereof; and alkenyl groups have 2 to 4 carbon atoms, for example vinyl.

In a compound of formula (I) the bonding of the divalent phenylene group may be *ortho*, *meta* or *para*, and in these variants any oxa or thia group is preferably adjacent the phenylene. When $X^1$ is other than phenylene, then $X^2$ may be $-CH_2-O-CH_2-$ or $-CH_2-S-CH_2-$.

Included in the scope of compounds of formula (I) are the salts corresponding to the carboxylic acids and tetrazoles when $X^3$ is carboxyl or tetrazolyl respectively,

and the salts which may also be formed when Z is hydrogen. Particularly valuable salts for medical purposes are those having a pharmaceutically acceptable cation such as ammonium or an alkali metal e.g.

BAD ORIGINAL

sodium and potassium, an alkaline earth metal e.g. calcium and magnesium, or an organic base, particularly an amine such as ethanolamine.

Salts having non-pharmaceutically acceptable cations are included within the ambit of this invention as useful intermediates to pharmaceutically acceptable salts, or the acids or esters of formula (I).

Compounds of formula (I) wherein Z is hydrogen or alkyl having 1 to 4 carbon atoms, for example methyl or butyl;

X is $-CH_2-$, $X^1$ is $-CH_2CH_2-$ or $-CH{:}CH-$, $X^2$ is alkylene of 1 to 5 in particular 3 carbon atoms, and $X^3$ is alkoxycarbonyl, carboxyl or a salt thereof;

R, $R^1$ and Y are as hereinbefore defined and $Y^1$ is hydrogen, phenyl, benzyl, or cycloalkyl of 4 to 7 carbon atoms;

have particularly interesting prostaglandin-like properties. Within this definition are included the subclass wherein Z is hydrogen.

The 5-carbon atom of the hydantoin ring of hydantoins of formula (I) is asymmetric, and a further asymmetric centre is present at 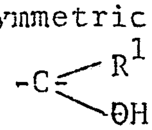. Compounds of formula (I) therefore exist as four isomers of formulae (IA), (IB), (IC) and (ID):

BAD ORIGINAL

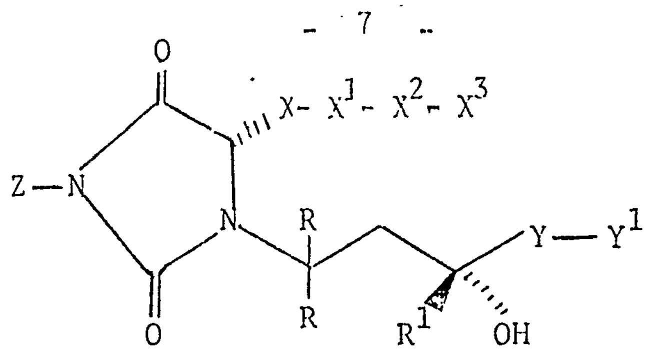

(IA)

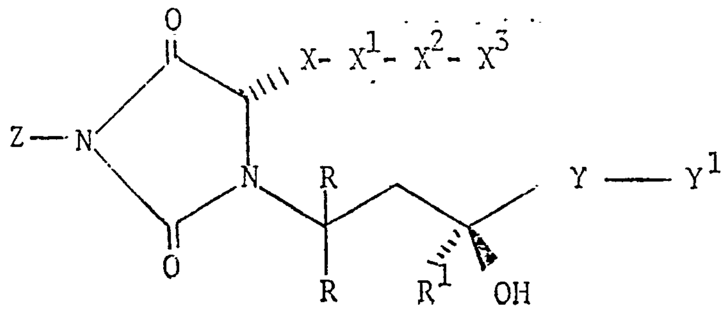

(IB)

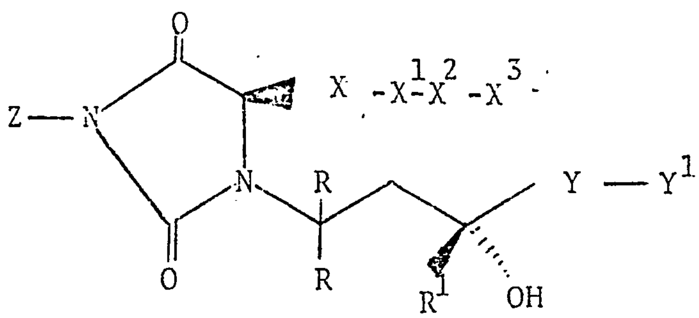

(IC)

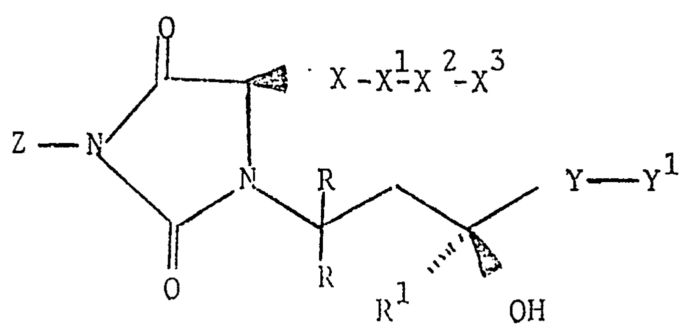

(ID)

wherein Z, X, $X^1$, $X^2$, $X^3$, R, $R^1$, Y and $Y^1$ are as defined in formula (I).

Hydantoins of formula (I) are separable by thin layer chromatography or high performance liquid chromatography (HPLC) into two diastereomers, each of which is a racemic mixture of two enantiomers namely, a mixture of an enantiomer of formula (IA) and an enantiomer of formula (ID), and a mixture of an enantiomer of formula (IB) and an enantiomer of formula (IC). On separation of the diastereomers, one diastereomer may be converted to a mixture of the two diastereomers by treatment with a base, such as an alkali metal hydroxide, and subsequently re-separated to provide two diastereomers. Repeated use of this technique enables the effectual conversion of one diastereomer to the other. Specific examples of the separation of diastereomers of compounds of formula (I) are given in our above-named co-pending applications.

It is also desirable to resolve one or both of the individual diastereomers into their enantiomers, since it is known to persons skilled in the art that the larger part of the biological activity of any racemic mixture is often attributable to one of the pair of enantiomers comprising the racemic mixture. The applicants, in attempting to effect such a resolution by using conventional resolving agents, found that the process proved difficult in view of the distance between the optically active centres and the carboxyl group.

BAD ORIGINAL

In order to solve the problems encountered during these experiments to resolve the enantiomers, the applicants attempted to resolve a racemic diester of formula (II):

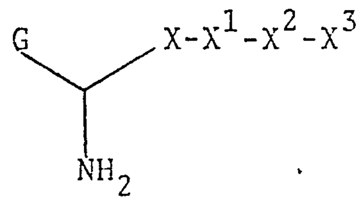

(II)

wherein X, $X^1$ and $X^2$ are as defined in formula (I), $X^3$ and G are the same or different and each is alkoxycarbonyl for example ethoxycarbonyl. At first, conventional methods were used, for example (+)-tartaric acid or dibenzoyl-(+)-tartaric acid were used as resolving agents; but this, too, proved unsuccessful.

Therefore, until the making of the present invention, a successful method for the resolution of the two enantiomers of one or, optionally, both of the diastereomers of compounds of Formula (I) had not been found.

Referring to Scheme A, it has now unexpectedly been found that a racemic diester of formula (II) undergoes selective hydrolysis on leaving an aqueous solution of a compound of formula (II) to

BAD ORIGINAL

stand at room temperature for several days, or by heating for several hours, in the presence of a carboxylic acid, for example acetic acid, to afford a racemic monoester of formula (III) wherein X, $X^1$, $X^2$ and $X^3$ are as defined in formula (II) and $G^1$ is carboxyl.

Subsequent acyclation by a standard method known in the art, for example by reaction with an acid halide such as acetyl chloride or, preferably, an acid anhydride such as acetic anhydride in an inert solvent, affords a racemic acylated monoester of formula (IV) wherein X, $X^1$, $X^2$, $X^3$ and $G^1$ are as defined in formula (III), $R^2$ is $(CH_2)_nQ$ wherein n is 1, 2 or 3 and Q is hydrogen or halo, for example chloro.

Selective deacylation, in particular deacetylation, using a suitable agent such as an enzyme affords a compound of formula (IIIA) wherein X, $X^1$, $X^2$, $X^3$ and $G^1$ are as defined in formula (III), and a compound of formula (IVB) wherein X, $X^1$, $X^2$, $X^3$, $G^1$ and $R^2$ are as defined in formula (IV). In particular, it has been found that selective deactylation using porcine renal acylase I in aqueous solution at neutral or slightly alkaline pH proceeds almost to completion. Separation of the species so formed may be effected by extraction of the acylated species into, for example, chloroform, the amino-acid remaining in the aqueous phase.

BAD ORIGINAL

More particularly, it has been surprisingly found that the selective deacetylation of a compound of formula (IV) which is 2-acetamidononanedioic acid 9-ethyl ester using porcine renal acylase I proceeds faster than would have been expected from reaction rates given in J P Greenstein and M Winitz, Chemistry of the Amino Acids Vol. 1, Wiley, New York, 1961 pages 744 ff.

BAD ORIGINAL

SCHEME A

A598

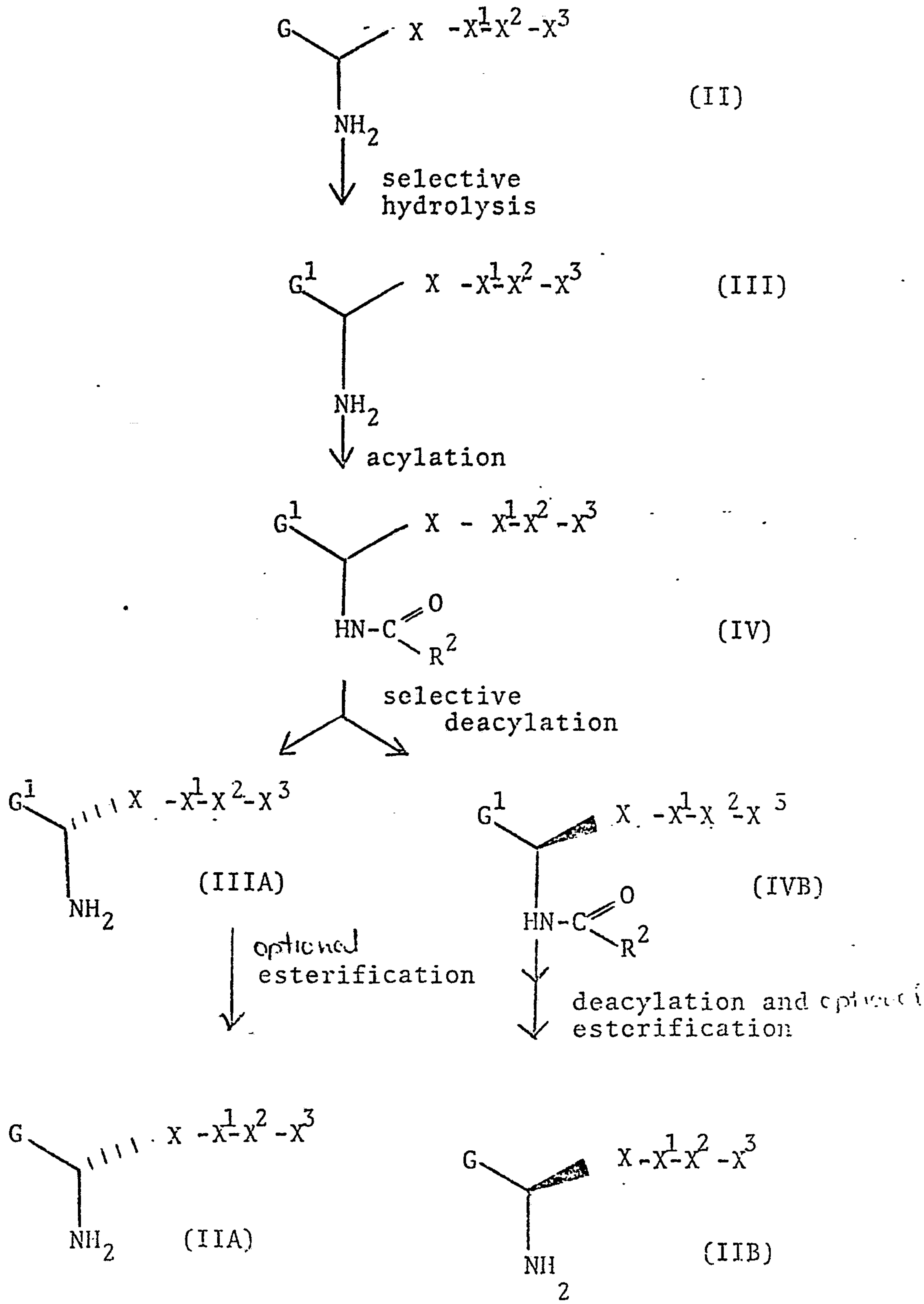


(II)

(III)

(IV)

(IIIA)

(IVB)

(IIA)

(IIB)

BAD ORIGINAL

The compound of formula (IIIA) is then optionally esterified using a standard method known in the art, for example, reaction with an appropriate alcohol such as ethanol and thionyl chloride to afford an amino-diester of formula (IIA) which is one of the enantiomers of the amino-diester of formula (II).

The compound of formula (IVB) may be optionally deacylated and re-esterified to afford an amino-diester of formula (IIB) which is the other enantiomer of the amino-diester of formula (II).

Preferred compounds of formula (II) for use in the present invention are those wherein

Z is hydrogen or alkyl;

X is methylene;

$X^1$ is $-CH=CH-$ or $-CH_2-CH_2-$;

$X^2$ is alkylene having 4 to 6 carbon atoms;

$X^3$ is alkoxycarbonyl such as ethoxycarbonyl;

Y is a covalent bond;

$Y^1$ is cycloalkyl of 4 to 7 carbon atoms

Each R is hydrogen; and

$R^1$ is hydrogen or methyl

Especially preferred are those wherein Z and $R^1$ are each hydrogen, for example, diethyl 2-aminononanedioate.

A preferred enantiomer of a compound of formula (II) is a compound of formula (IIA), for example diethyl (S)-2-aminononanedioate.

Where the selective deacylation of a compound of formula (IV) proceeds to completion of the

BAD ORIGINAL

reaction, the isomers of formula (IIA) or (IIB) finally produced would be 100% pure. Where the selective deacylation proceeds almost to completion, the isomer of formula (IIA) produced would be 100% pure while the isomer of formula (IIB) may be mixed with a small amount of that of formula (IIA).

The compounds of formula (IIA) may then be converted to the compounds of formula (I) by methods analogous to those described in our co-pending applications, as shown in Scheme B. Initially, the compound of formula (IIA) is converted to a mixture of epimers of formulae (VA) and (VB):

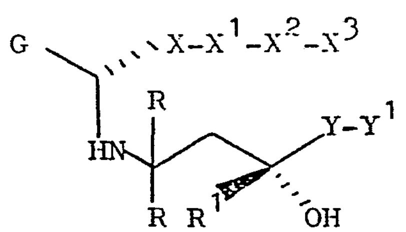 (VA) 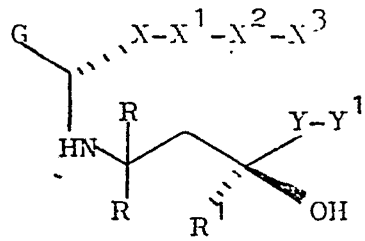 (VB)

which may then be separated, for example, by HPLC.

oreover, it will be understood that the re-esterification could have been effected at a later stage in the process, for example by esterifying the compound of formular(VIIA) or (VA). Hence, in the formulae of these compounds and accordingly of the intermediates for the synthesis of these compunds, G could be replaced by $G^1$. In this case, the preferred monoester of formula (IIIA) is (S)-2-aminononanedioic acid 9-ethyl ester.

BAD ORIGINAL

SCHEME B

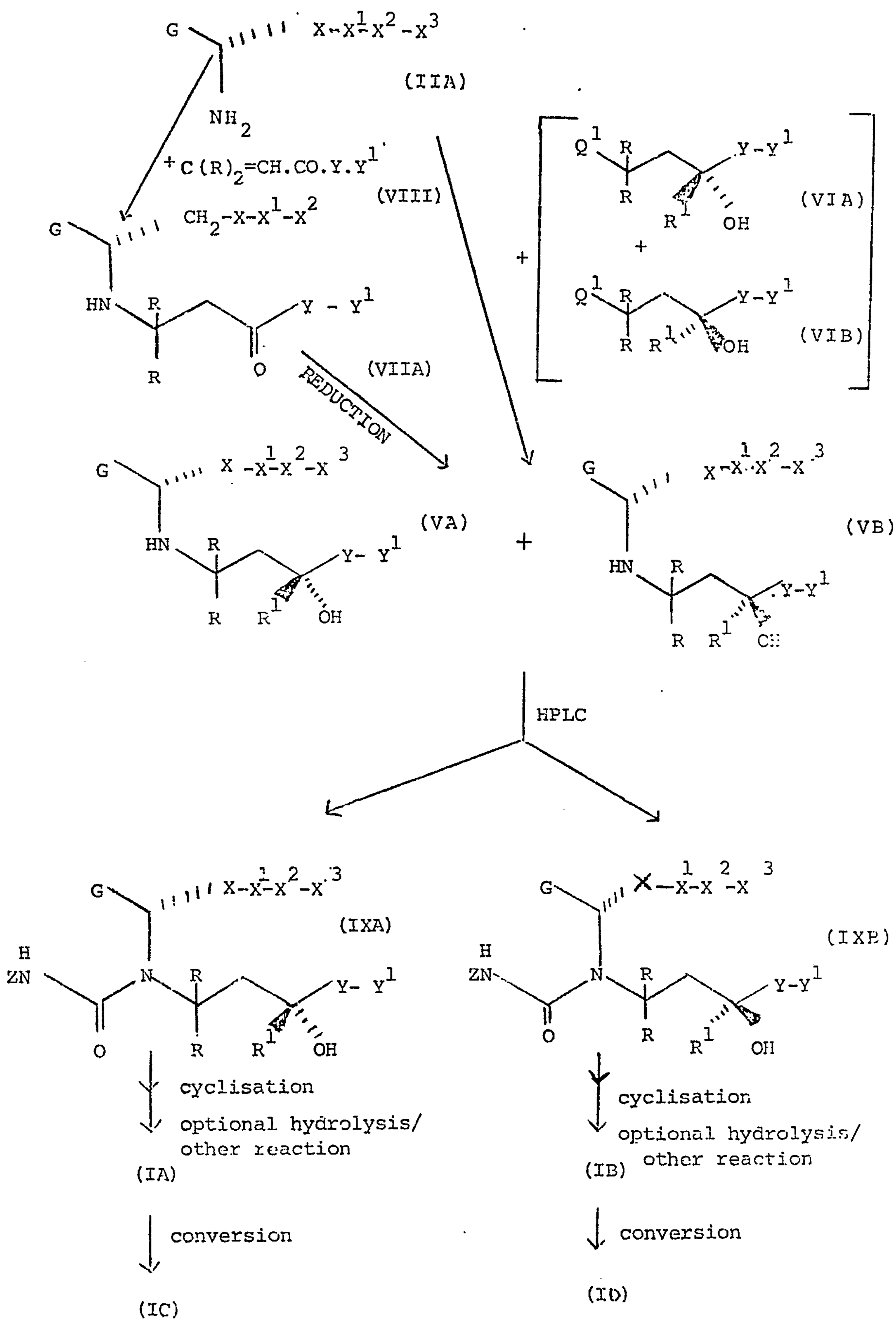


(IIA)

(VIII)

(VIA)

(VIB)

(VIIA)

(VA)

(VB)

(IXA)

(IXB)

(IA)

(IB)

(IC)

(ID)

BAD ORIGINAL

wherein G, X, $X^1$, $X^2$ and $X^3$ are as defined in formula (IIA) and R, $R^1$, Y and $Y^1$ and $Y^1$ are as defined in formula (I); by reaction of a compound of formula (IIA) with a racemic mixture of compounds of formulae (VIA) and (VIB):

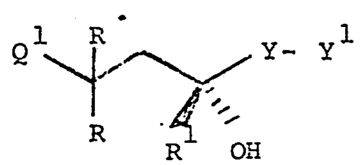

(VIA)

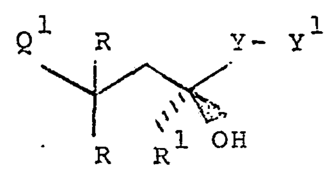

(VIB)

wherein R, $R^1$, Y and $Y^1$ are as defined in formulae (VA) and (VB), and $Q^1$ is halo, preferably bromo. The reaction may be carried out by heating in the absence of a solvent or in the presence of an inert solvent such as ethanol.

The mixture of compounds of formula (VA) with compounds of formula (VB) may also be prepared by reduction of a ketone of formula (VIIA);

BAD ORIGINAL

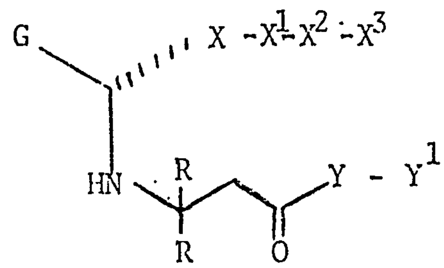

(VIIA)

wherein G, R, X, $X^1$, $X^2$, $X^3$, Y and $Y^1$ are as defined in formulae (VA) and (VB). The reduction may be effected by standard methods known in the art, for example with a metal borohydride.

The intermediates of formula (VIIA) may be prepared by reaction of an amine of formula (IIA) with an unsaturated ketone of formula (VIII):

$$C(R)_2 = CH.CO.Y.Y^1 \qquad \text{(VIII)}$$

wherein R, Y and $Y^1$ have the same meaning as in formula (VIIA); the reaction being effected in the presence or absence of an inert solvent, and at room temperature or optionally with cooling or heating.

A compound of formula (VA) or (VB) prepared as hereinbefore described may be used to prepare one of the optically active forms of a compound of formula (IX):

BAD ORIGINAL

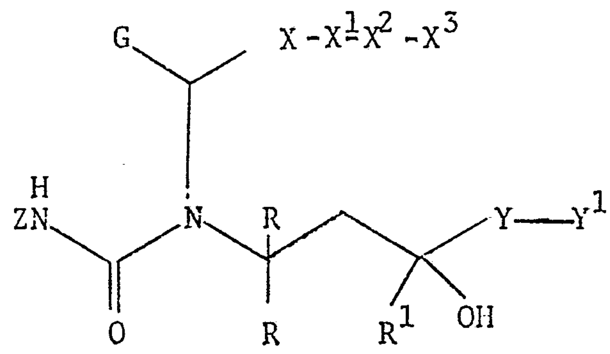

(IX)

wherein G, Z, X, $X^1$,$X^2$, $X^3$, R, $R^1$, Y and $Y^1$ are as previously defined, provided that G may also be nitrile; by reaction with cyanic acid or an alkyl *iso*-cyanate depending respectively on whether Z is hydrogen or alkyl.

When cyanic acid is used, the cyanic acid is conveniently produced *in situ* by the use of an alkali metal cyanate, e.g potassium cyanate, and an acid which may be present as an acid addition salt of the compound of formula (VA) or (VB) or a free acid of formula (VA) or (VB) wherein G is carboxyl. Alternatively an equivalent amount of mineral acid or organic acid may be added to the reaction medium. An inert solvent is used which is preferably polar such as water or a mixture of water with acetone, dimethylformamide, dimethylsulphoxide, or a lower alkanol such as ethanol, an ether, or halogenated hydrocarbon such as chloroform. Where desired, for

BAD ORIGINAL

example if no solvent is used, the reaction may be promoted by heating the reactants.

Similar reaction conditions may be used when an alkyl iso-cyanate is used except that it is unnecessary to provide an equivalent amount of acid, as an acid addition salt or otherwise, with the reactants.

Instead of using a cyanate or isocyanate, a compound of formula (VA) or (VB) may be reacted with urea, nitrourea or an N-alkylurea as appropriate. A solvent is not essential but if desired an inert solvent such as one mentioned above may be used, and the reaction is preferably effected at an elevated temperature, for example 100° to 125°C but temperatures up to 150°C may be employed.

Cyclisation of the compounds of formula (IXA) or (IXB), comprises one of the methods for synthesising compounds of formula (IA) or (IB), respectively, as may be found in the art for the synthesis of compounds of analogous structure; and, in particular, one of the methods for synthesising compounds of formula (I) as described in our abovementioned co-pending patent applications. The reaction may be effected in the absence of a solvent, but if

BAD ORIGINAL

desired an inert solvent may be used, for example a hydrocarbon such as petrol. Alternatively, where $G^1$ has been converted to the ester G, by standard methods as described above, cyclisation may be effected in the presence of a suitable base, for example an alkoxide such as sodium ethoxide. The compounds of formula (IA) or (IB) may then be optionally converted into the compounds of formula (IC) or (ID), respectively, by treatment with aqueous sodium hydroxide and separation of the resulting mixture of epimers with HPLC.

The present invention therefore provides the preparation of pure or substantially pure (as hereinafter defined) compounds of formula (IA) and (IB). Thus, after optional mild hydrolysis of a compound of formula (IA) or (IB) (for example, to convert a compound wherein $X^3$ is alkoxycarbonyl to a compound wherein $X^3$ is carboxyl) the compound of formula (IA) or (IB) so produced may be mixed with a small amount of the epimer of formula (IC) or (ID), respectively, in a ratio of not less than about 95:5 w/w. Therefore, by 'substantially pure' or 'in substantially pure form' is meant not less than 95% by weight of the stated compound.

Further methods for preparing compounds of formula (IA), (IB), (IC) or (ID) are set out below by referring to the preparation of the compounds of formula (IA) and (IB). The analogous processes for preparing the compounds of formula (IC) and (ID) are

BAD ORIGINAL

to be implied hereinbelow.

Hydantoins of formula (IA) or (IB) may be prepared by cyclisation of a compound of formula (XA) or (XB) respectively:

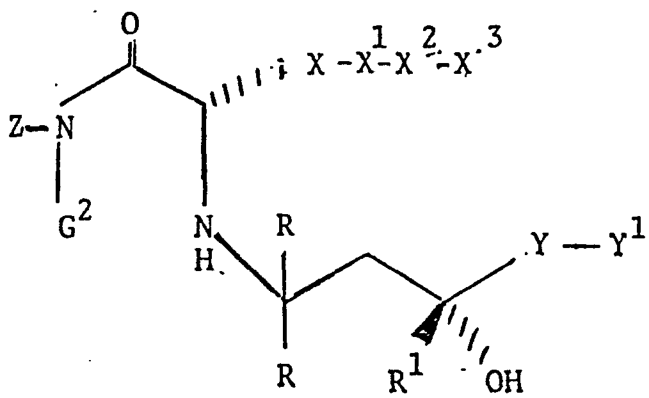

(XA)

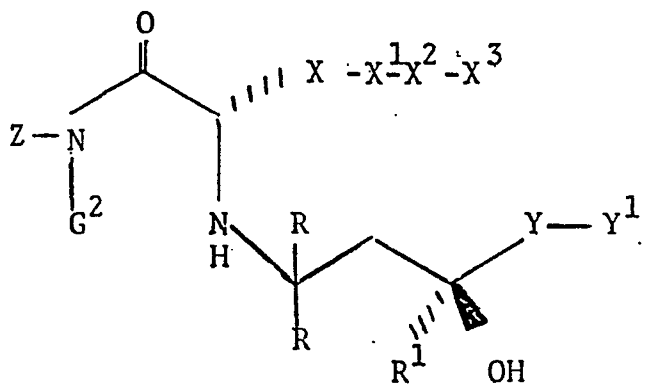

(XB)

wherein Z, X, $X^1$, $X^2$, $X^3$ R, $R^1$, Y and $Y^1$ are as defined in formula (IA) or (IB), respectively, and $G^2$ is carboxyl or a reactive derivative thereof such as alkoxycarbonyl e.g. ethoxycarbonyl. Compounds of formula (XA) and (XB) may be cyclised under similar conditions as a compound of formula (IXA) or (IXB) and conveniently the method used to prepare a compound of formula (XA) or (XB) is chosen such that the prevailing reaction conditions permit spontaneous cyclisation. Optional conversion into hydantoins of

formula (IC) or (ID) as hereinbefore described may follow.

The intermediates of formula (XA) or (XB) may be prepared, for example, by reacting a compound of formula (VIA) or (VIB), respectively, with a compound of formula (XIA)

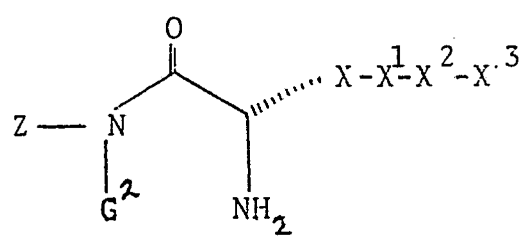

(XIA)

wherein $G^2$, Z, X, $X^1$, $X^2$ and $X^3$ have the same meaning as in formula (XA) or (XB), and in formula (VIA) or (VIB) $Q^1$ is halogeno, preferably chloro or bromo. The reaction may be effected by admixture of the reactants or optionally an inert solvent is used and the mixture is heated. Suitable solvents include alkanols, ethers, hydrocarbons and halogenated hydrocarbons. The diastereomers so formed may be separated by HPLC.

The compounds of formula (XIA) may themselves be made by reacting an appropriate carbamic acid derivative, for example an alkyl ester, with a compound of formula (IIA), using a technique known to

BAD ORIGINAL

those skilled in the art.

In a method related to those described hereinbefore, the hydantoins of formula (IA) or (IB) may be prepared by reacting a compound of formula (XIIA) or (XIIB):

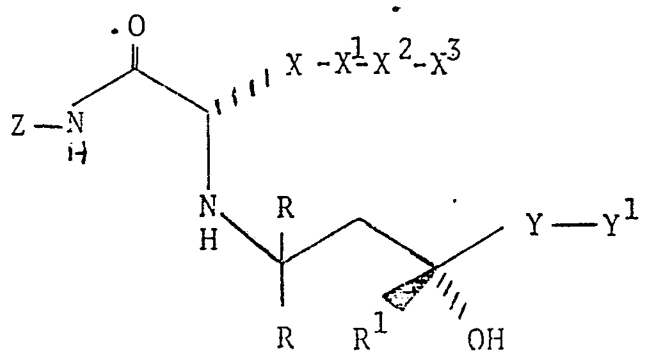

(XIIA)

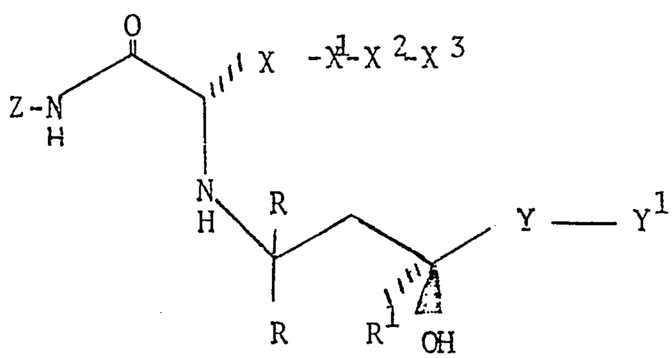

(XIIB)

wherein each of Z, R, $R^1$, X, $X^1$, $X^2$, $X^3$, Y and $Y^1$ has the same meaning as in formula (IA) or (IB), respectively, with a carbonic acid derivative. Any carbonic acid derivative known to those skilled in the art as appropriate may be used, for example phosgene, diphenylcarbonate or an alkyl haloformate such as

BAD ORIGINAL

ethyl chloroformate. The reaction is desirably effected in the presence of a base, for example an amine such as triethylamine or di-iso-propyl ethylamine, and using an inert aprotic solvent such as toluene, dimethylformamide or an ether such as diethyl ether. The reaction may be carried out at room temperature but if desired the reaction mixture may be heated. Optional conversion into hydantoins of formula (IC) or (ID) as hereinbefore described may follow.

The intermediates of formula (XIIA) or (XIIB) may be made using methods analogous to those described above for the preparation of compounds of formula (VA) or (VB), respectively.

The four isomers of hydantoins of formula (I) wherein Z is alkyl may also be prepared by alkylation, using an alkylating agent which may be designated as a reactive ester derivative of an alcohol $J^3.OH$ of the corresponding isomer of a compound of formula (XIII) and separating the resulting mixture of isomers, for example by HPLC.

BAD ORIGINAL

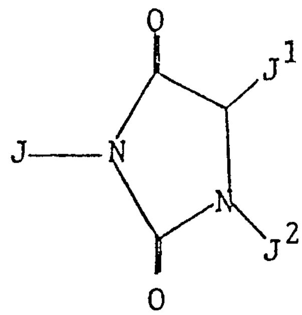

(XIII)

In formula (XIII)

$J^3$ has the same meaning as Z

$J^1$ is $-X-X^1-X^2-X^3$, J is hydrogen and

$J^2$ is $-C(\underline{R})_2-CH_2-C(R^1)(OH)-Y-Y^1$

Suitable reactive ester derivatives include chloride, bromide, iodide and sulphonates such as p-toluenesulphonate, methanesulphonate and benzenesulphonate. The alkylation may be effected using reaction conditions which are known in the art to be suitable, for example in the presence of a base such an alkali metal hydride, alkali metal amide, or alkali metal alkoxide, typically sodium hydride or a sodium alkoxide e.g. sodium methoxide.

The reaction is conveniently carried out in an inert solvent which simply acts as a diluent for the reactants such as toluene, dioxan, ether, dimethylformamide, tetra-

hydrofuran, dimethylsuplphoxide or acetonitrile or when the base is an alkali metal alkoxide then the corresponding alkanol may be used.

It will be appreciated that the intermediates of formula (XI) wherein J is hydrogen are also compounds of formula (I) and may be prepared by one of the foregoing methods. The compounds of formula (XI) may further be prepared by adaptation of methods already known in the art (see for example Chemical Reviews (1950) 46 p 403-425).

The alcohols of any of the isomers of hydantoins of formula (I) wherein $X^3$ is hydroxymethyl may also be obtained by reduction with an appropriate reducing agent of the corresponding acid, ester, acid halide, acid anhydride or aldehyde. The appropriate reducing agent will depend on the particular substrate, but a reactant which may be used is sodium in ethanol. In particular, a carboxylic acid may, for example, be converted to a corresponding mixed anhydride with ethyl chloroformate in the presence of a base such as triethylamine, and subsequently reduced to the alcohol using sodium borohydride. Similarly an ester may be reduced to the alcohol using di-*iso*-butyl aluminium hydride in an inert solvent such as ether or hydrocarbon such as hexane or benzene. Such alcohols may also be prepared by catalytic hydrogenation.

BAD ORIGINAL

Alternatively the isomers of alcohols of formula (I) wherein $X^3$ is hydroxymethyl may be prepared by hydrolysis of a corresponding $X^3$ halide with an appro - priate reagent. For this purpose a hydroxide may be used for example an aqueous alkali or a suspension of silver oxide in water.

In the synthesis of any of the isomers of hydantoins of formula (I) it may be desirable to protect the hydroxyl group in the side chain during the course of the reaction. This may be readily effected in a known manner using a protecting group such as acyl, aroyl, tetrahydropyran-2-yl, 1-ethoxy-ethyl or aralkyl, for example benzyl.

Removal of protecting groups may be carried out by appropriate methods known to those skilled in the art: for example an acyl group may be removed by acid or base hydrolysis, and a benzyl group by reductive cleavage.

Where the compounds of formula (I) have a C≡C or CH=CH bond these may be converted by conventional hydrogenation techniques, for example using a Lindlar type or Adams catalyst, to the corresponding ethylenic or saturated compounds as appropriate.

BAD ORIGINAL

In all of the foregoing chemical procedures it is of course evident that the choice of reactant will be dictated in part by the functional groups present in the substrate, and where necessary reactants having an appropriate selectivity of action must be used.

The hydantoins of formula (I) are of value in having pharmacological properties related to those of natural prostaglandins; as described in our applications listed above.

A group of compounds which are particularly valuable as inhibitors of platelet aggregation are those of formula (I) wherein Z is hydrogen, X is $-CH_2-$, $X^1$ is

BAD ORIGINAL

$-CH_2.CH_2-$ or $-CH:CH-$, $X^2$ is alkylene optionally including an oxa or thia group, $X^3$ is -COOH and Y is a bond or branched alkylene having a tertiary carbon atom adjacent to $-C(R^1)(OH)-$ and $Y^1$ is cycloalkyl.

Within this group of compounds, are those wherein $-CH_2-X^1-X^2-COOH$ is carboxyhexyl, carboxymethoxybutyl, carboxymethylthiobutyl and the corresponding unsaturated radicals where $X^1$ is -CH:CH-, Y is a bond and $Y^1$ is cyclopentyl or cyclohexyl. Particular compounds which may be mentioned for their anti-platelet-aggregatory properties are:

5(6-carboxyhexyl)-1-(3-cyclohexyl-3-hydroxypropyl) hydantoin, 5-(4-carboxymethoxybutyl)-1-(3-cyclohexyl-3-hydroxypropyl) hydantoin, 5-(4-carboxymethylthiobutyl)-1-(3-cyclohexyl-3-hydroxypropyl)hydantoin, 5-(6-carboxyhex-2_Z_-enyl)-1-(3-cyclohexyl-3-hydroxypropyl)hydantoin and 5-(4-carboxymethoxybut-2_Z_-enyl)-1-(3-cyclohexyl-3-hydroxypropyl)hydantoin.

The hereinbefore described methods of preparing the individual optical isomers of compounds of formula (I), have made possible the investigation of their biological properties. It has been found, as might have been expected, that one of the optical isomers in each diastereomer

BAD ORIGINAL

of one of the compounds of formula (I) named above, namely, 5-(6-carboxyhexyl)-1-(3-cyclohexyl-3-hydroxypropyl)hydantoin is a greater inhibitor of platelet aggregation than the other. That is, the corresponding compound of formula (IA) is more active than the corresponding compound of formula(ID) and the corresponding compound of formula (IB) is more active than the corresponding compound of formula (IC).

However, it has unexpectedly been found that, in the case of 5-(6-carboxyhexyl)-1-(3-cyclohexyl-3-hydroxypropyl) hydantoin, the isomer corresponding to that of formula (IA), namely, (5S)-5-(6-carboxyhexyl)-1-[(3R)-3-cyclohexyl-3-hydroxypropyl] hydantoin has an anti-platelet-aggregation inhibitory effect which is at least twice that of the activity of the corresponding racemate, while its mirror-image, namely, (5R)-5-(6-carboxyhexyl)-1-[(3S)-3-cyclohexyl-3-hydroxypropyl] hydantoin is inactive. Specifically, the activity of the racemate (a mixture of the (5S,3R) and the (5R,3S) isomers) is in the range of from 12 to 16 times that of $PGE_1$, while the activity of the (5S, 3R) enantiomer is of the order of 40 times that of $PGE_1$. The (5S,3S) and (5R, 3R) isomers have an activity of 0.06 and 0.05 times that of $PGE_1$, respectively.

BAD ORIGINAL

The applicants believe that, provided that the configuration at the two optical centres remain constant, a similar effect could be found for all compounds of formula (I) such that compounds of formula (IA) are always at least twice as active as a mixture of the corresponding compounds of formulae (IA) and (ID); while the isomers of formulae (IB) and (IC) are only slightly active.

Other medicinal uses to which compounds of formula (I) may be put are more fully explained in our earlier applications, (the disclosures of which are to be understood to be incorporated herein by reference), together with modes of administration and pharmaceutical compositions therefor.

In addition to the above described effects at least some of the compounds of formula (I), notably compounds of formula (IA), also exhibit a vasodilatory action on blood vessels, as described in the complete specification of our co-pending UK patent application No. 20694/78. There are often occasions when it would be desirable to be able to utilise the anti-aggregatory effects and the other effects described in our earlier applications while at the same time suppressing or eliminating the vasodilatory effect of the isomers of compounds of formula (I), for

BAD ORIGINAL

example in the treatment or prevention of myocardial infarcts and when used as an addition in extra-corporeal circulation.

The anti-aggregatory effects, but not the vasodilatory effects, of the active compounds are potentiated by chemical compounds exhibiting a particular pharmacological activity. This provides a means to dissociate the anti-aggregatory effects from the vasodilatory effects of the compounds of formula (I) and notably the compounds of formula (IA).

Accordingly, a pharmaceutical combination which comprises an optical isomer of a compound of formula (I), notably a compound of formula (IA) and a phosphodiesterase inhibitor promotes an anti-aggregatory action on blood platelets.

The use, in combination, of an optical isomer of a compound of formula (I), notably a compound of formula (IA), and a phosphodiesterase inhibitor, lowers the anti-aggregatory threshold level (the minimum amount of active compound required to exhibit anti-aggregatory effects) without substantially affecting the vasodilatory threshold level.

BAD ORIGINAL

Thus, by administering an active compound at a concentration below its vasodilatory threshold level, but above the new, lower, anti-aggregatory threshold, together with a phosphodiesterase inhibitor, anti-aggregatory effects may be produced whilst vasodilatory effects are not.

Suitable phosphodiesterase inhibitors for use in potentiating the anti-aggregatory effects of the active compounds include:-

Xanthine derivatives such as:

Theophylline (3,7-dihydro-1,3-dimethyl-1*H*-purine-2,6-dione), and salts thereof;

3-Isobutyl-1-methyl-xanthine;

Caffeine (3,7-dihydro-1,3,7-trimethyl-1*H*-purine-2,6-dione), and salts thereof; and

Aminophylline (adduct of Theophylline and 1,2 - ethanediamine (2:1)).

Phosphodiesterase inhibitors other than xanthine derivatives that may be used in combination with optical isomers provided by the present invention include, as such or as pharmaceutically acceptable salts:

(a) Isoquinoline derivatives, for example:

Papaverine (1-[(3,4-dimethoxyphenyl)methyl]-6,7-dimethoxyisoquinoline), and salts thereof; and

BAD ORIGINAL

6,7-Diethoxy-1-(4,5-diethoxybenzyl)isoquinoline, or its salts e.g. its hydrochloride;

(b) Derivatives of pyrimido (5,4-d)pyrimidine, for example:

Dipyridamole (2,2',2",2'"-(4,8-dipiperidinopyrimido [5,4-d] pyrimidine-2,6-diyldinitrilo)tetraethanol) and its salts;

2,2',2",2'",- [[4-(1-piperidinyl)pyrimido [5,4-d] pyrimidin-2,6-diyl] dinitrilo] tetrakisethanol and its salts; and

2,4,6-tri-4-morpholinylpyrimido [5,4-d] pyrimidine and its salts;

(c) Derivatives of thieno [3,2-d] pyrimidine, for example:

N- [4-(4-morpholinyl)thieno [3,2-d] pyrimidin-2-yl] -1,2-ethanediamine;

(d) Derivatives of pyrazolo[3',4':2,3] pyrido-[4,5-b] [1,5] benzodiazepin-6-(3H)- one, for example:

3-Ethyl-7,12-dihydro-7, 12-dimethylpyrazolo-[4',3':5,6] pyrido [4,3-b] - [1,5] benzodiazepin-6-(3H)-one;

3-Ethyl-7,12-dihydro-9-methoxy-7, 12-dimethyl-pyrazolo[3',4':2,3]pyrido [4,5-b][1,5] benzodiazepin-6-(3H)- one; and

BAD ORIGINAL

10-Chloro-3-ethyl-7, 12-dimethyl-7, 12-dihydro pyrazolo [4',3':5,6]pyrido [4,3-b] [1,5] benzodiazepin-6-(3H)-one;

(e) Derivatives of 1H- or 2H-pyrazolo [3,4-b] pyridine, for example:

4-(Butylamino)-1-ethyl-1H-pyrazolo[3,4-b] pyridine-5-carboxylic acid ethyl ester;

4-(Butylamino)-1H-pyrazolo [3,4-b]pyridine-6-carboxylic acid ethyl ester;

4-Chloro-1-ethyl-3-methyl-1H-pyrazolo[3,4-b] -pyrimidine-5-acetonitrile;

1-Ethyl-4-(isopropylidenehydrazino)-3-methyl-1H-pyrazolo[3,4-b] pyridine-5-carboxylic acid ethyl ester or its salts such as its hydrochloride hemihydrate; and

2-Methyl-6-phenyl-4-(1-piperidinyl)-2H-pyrazolo [3,4-b] pyridine or its salts e.g. its hydrochloride;

(f) Derivatives of 5H-furo-[3,4-c]pyrazolo[3,4-b] pyridine-5-one, for example:

4-(Butylamino)-1-ethyl-1, 7-dihydro-7-hydroxy-5H-furo-[3,4-e] pyrazolo [3,4-b]pyridine-5-one; and

(g) Derivatives of 1-(2H)-naphthalenone, for example:

2 [(Dimethylamino)methyl] -3,4-dihydro-7-methoxy-1-(2H)naphthalenone or its salts e.g its 1:1 hydro-

BAD ORIGINAL

hydrochloride.

A particularly preferred combination is one comprising (5S)-5-(6-carboxyhexyl)-1-[(3R)-3-cyclohexyl-3-hydroxypropyl] hydantoin as compound of formula (IA) and dipyridamole as phosphodiesterase inhibitor.

In this combination the dipyridamole has produced a potentation of the anti-aggregatory effect of the active compound in aggregation tests on rabbits and in human platelet rich plasma.

The amount of a compound of formula (I) required to achieve the desired biological effect will of course depend on a number of factors including the use for which it is intended, the mode of administration, the recipient and the specific compound chosen. Examples of such doses are also given in our earlier applications but

having regard to the largely increased activity of (5S)-5-(6-carboxyhexyl)-1-[(3R)-3-cyclohexyl-3-hydroxypropyl] hydantoin over that expected and, hence, over that envisaged to be within the scope of our earlier applications, the appropriate dose for such active isomers would be reduced by a corresponding factor.

BAD ORIGINAL

More specifically, when such a compound of formula (IA) is used to inhibit platelet aggregation it is considered desirable to achieve a concentration in the appropriate liquid, whether it be the blood of a patient or a perfusion fluid, in the range of from 0.25 μg. to 2.5mg for example in the range of from 2.5 μg to 0.25 mg per litre.

The amount of isomer of a compound of formula (I) in such a combination required for therapeutic effect will vary not only with the particular compound and the route of administration but also with the particular phosphodiesterase inhibitor used and its amount, i.e. with the degree of potentiation that occurs. The amount of the isomer will, however, be below that required to produce a given therapeutic effect in the absence of the potentiator and thus also below the level which would produce a significant vasodilatory effect.

Where a phosphodiesterase inhibitor is used a suitable dose of compound of formula (I) will in general be half or, say, up to about 75% of the dose in the absence of phosphodiesterase inhibitor potentiator.

BAD ORIGINAL

In general the compound of formula (I) and phosphodiesterase inhibitor are administered in a weight ratio of 1 part of the compound to from 1 to 200, preferably from 10 to 150, especially 40 to 100, e.g. 60, parts of phosphodiesterase inhibitor.

While it is possible for an isomer of a compound of formula (I), notably, a compound of formula (IA), and a phosphodiesterase inhibitor to be administered as raw chemicals, it is preferable to present them as one or more pharmaceutical formulations.

More specifically, when such a combination contains a compound of formula (IA) and is presented as a pharmaceutical formulation, a unit dose of such a formulation may contain, for example, 75 mg of phosphodiesterase inhibitor and 1.25 mg of the compound of formula (IA).

The abovementioned doses of the active compounds refer to the acids, amides, esters, alcohols and tetrazoles of formula (I); where a salt is used, the dose should be taken as referring to the corresponding anion.

It will be appreciated from the foregoing that what we shall claim may comprise any novel feature

BAD ORIGINAL

described herein, but principally and not exclusively, for example:-

(a) a method for preparing four individual isomers of formula (I), namely compounds of formula (IA), (IB), (IC) or (ID) as hereinabove described;

(b) a compound of formula (IA), (IIA), (IIIA) or (IXA) as hereinbefore defined when prepared by the method as hereinabove described;

(c) a pharmaceutical formulation comprising a compound of formula (IA) in association with a pharmaceutically acceptable carrier therefor, and methods for the preparation of such formulations;

(d) a method for inhibiting platelet aggregation in a mammal which comprises administration to said mammal of a non-toxic, effective, inhibitory amount of a compound of formula (IA)

(e) a pharmaceutical formulation comprising a compound of formula (IA) and a phosphodiesterase inhibitor in association with a pharmaceutically acceptable carrier therefor, and methods for the preparation of such formulations;

(f) a method for inhibiting platelet aggregation in a mammal which comprises administration to

BAD ORIGINAL

said mammal of a non-toxic, effective inhibitory amount of a compound of formula (IA) and a phosphodiesterase inhibitor;

(g) A method of lowering blood pressure in a mammal which comprises administration to said mammal of a non-toxic, effective, hypotensive amount of a compound of formula (IA);

(h) A method for inducing vasodilation in a mammal which comprises administration to said mammal of a non-toxic, effective vasodilatory amount of a compound of formula (IA); and

(i) A method for inducing bronchodilation in a mammal which comprises administration to said mammal of a non-toxic effective bronchodilatory amount of a compound of formula (IA).

The following Examples are provided by way of illustration of the present invention and should not be construed as in any way constituting a limitation thereof. All temperatures are in degrees Celsius.

BAD ORIGINAL

Example 1 preparation of the four optical isomers of 5-(6-carboxyhexyl)-1-(3-cyclohexyl-3-hydroxy propyl)hydantoin.

A. Preparation of diethyl (S)-2-aminononanedioate

Diethyl 2-aminononaedioate (50g) was heated with water (100ml) and acetic acid (6ml) on a steam bath for 6 hours and the resulting suspension of colourless crystals was set aside at room temperature overnight. It was then filtered, affording 2-amino-nonanedioic acid 9-ethyl ester (26g), m.p. 214-216$^{\circ}$ (efferv.). Concentration of the liquors to 30ml and heating for 2.5 hours gave a further 10.1g of the same product.

A well-stirred suspension of 2-aminononanedioic acid 9-ethyl ester (26g) in acetic acid (78 ml) was treated dropwise with acetic anhydride (12.7 ml) for 1 hour. After a further 3 hours, the reaction solution was stirred with water (250 ml) and ether (250ml) for 1 hour, and the mixture was then shaken with more water and ether. The ethereal phase was separated, washed with water, dried ($MgSO_4$), and evaporated; the residual oil was freed from traces of acetic acid by distillation with carbon tetrachloride in vacuo and finally set aside in ether (50 ml) at 0$^{\circ}$, to give 2-acetamidononanedioic acid 9-ethyl ester (19.1g), mp. 84-85.5$^{\circ}$.

BAD ORIGINAL

A stirred suspension of 2-acetamidononanedioic acid 9-ethyl ester (12.95 g) in water (390 ml) was treated dropwise with concentrated aqueous ammonia to pH 7-7.5 and the solution was filtered from sediment and diluted with water (97 ml). Porcine renal acylase I (0.162 g) and cobaltous acetate tetrahydrate (0.615 g) were added and the mixture was kept in a bath at 37° for 2 days. After cooling to room temperature and filtration from sediment, the aqueous solution was treated with more acylase I (0.162 g) and maintained at 37° for an additional 2 days. The reaction mixture was treated with hydrogen sulphide and filtered from cobalt sulphide, and the aqueous solution was brought to pH 4 with acetic acid, washed with chloroform (7 x 900 ml), and evaporated *in vacuo*. The residual crystals were freed from traces of water by distillation with ethanol *in vacuo*, suspended in ethanol (ca. 10 ml), and collected, to give (S)-2-aminononanedioic acid 9-ethyl ester (4.53 g), m.p 235-237° (efferv.), $[\alpha]_D^{25}$ +23.5° (c = 1 in HOAc).

Ethanol (16.3 ml) was stirred at -15° and treated dropwise with thionyl chloride (1.71 ml), and (S)-2-aminononanedioic acid 9-ethyl ester (2.5 g) was then added in portions, keeping the temperature at - 10°.

BAD ORIGINAL

The stirred suspension was allowed to come to room temperature and the resulting solution was set aside at room temperature for 16 hours and poured on to an ice-water mixture containing concentrated aqueous ammonia (6.2 ml). The product was extracted into ether, the ethereal solution was concentrated *in vacuo* to *ca.* 50 ml, water (125 ml) was added, and the ether and water were evaporated *in vacuo*. The residual oil was freed from traces of ethyl sulphite by dispersion in water (2 x 125 ml) and evaporation of the water *in vacuo*, to leave diethyl (S)-2-aminononanedioate (2.5g) as a colourless oil, $[\alpha]^{21}_{D}$ + 16.3° (c = 1.02 in EtOH).

BAD ORIGINAL

B. Preparation of diethyl (2S)-2-[(3R)-3-cyclohexyl-3-hydroxypropylamino]nonanedioate.

To diethyl (S)-2-aminononanedioate (2.2g) was added dropwise cyclohexyl vinyl ketone (1.23g) and the mixture was allowed to stand at room temperature for 18 hours to give diethyl (2S)-2-(3-cyclohexyl-3-oxopropylamino)nonanedioate.

The foregoing crude ketone was stirred in ethanol (35 ml) and treated with sodium borohydride (0.242 mg). After 2.5 hours the alcohol was evaporated in vacuo and the residue was cooled in ice-water. The insoluble oil was extracted with ether, and the ether solution was washed with water, dried ($MgSO_4$) and evaporated to leave a mixture of diethyl (2S)-2-[(3R)-3-cyclohexyl-3-hydroxypropylamino]-nonanedioate, and diethyl (2S)-2-[(3S)-3-cyclohexyl-3-hydroxypropylamino] nonanedioate.

Separation by high performance liquid chromatography (HPLC) on "Biosil", using chloroform-methanol mixtures as eluant, afforded diethyl (2S)-2-[(3R)-3-cyclohexyl-3-hydroxypropylamino]nonanedioate (1.1g),

BAD ORIGINAL

$[\alpha]_D^{22}$ +1.09° (c = 0.97 in EtOH) and diethyl (2S)-2-[(3S)-3-cyclohexyl-3-hydroxypropylamino] nonanedioate (0.96g), $[\alpha]_D^{22}$ - 7.26° (c = 1.01 in EtOH), both as colourless gums.

C. Preparation of (5S)-5-(6-carboxyhexyl)-1-[(3R)-3-cyclohexyl-3-hydroxypropyl] hydantoin and (5S)-5-(6-carboxyhexyl)-1- [(3S)-3-cyclohexyl-3-hydroxypropyl] hydantoin.

A stirred solution of diethyl (2S)-2-[(3R)-3-cyclohexyl-3-hydroxypropylamino] nonanedioate (1.1 g) in ethanol (5.5 ml) was cooled in ice-water and treated with 2N-hydrochloric acid (2.76 ml) followed by a solution of potassium cyanate (0.447 g) in water (1.35 ml). The turbid solution was set aside at room temperature overnight, the ethanol was evaporated *in vacuo*, and the residue was shaken with water and ether. The ethereal phase was separated, washed with water, dried ($MgSO_4$), and evaporated, to give an oil which was heated at 100° for 8.5 hours. The oil was stirred with 0.5N-aqueous sodium hydroxide (11 ml) at room temperature for 55 minutes and the turbid reaction solution was shaken with water (30 ml) and ether (30 ml); the aqueous phase was separated, washed with ether and acidified with N-hydrochloric acid, and

BAD ORIGINAL

the liberated carboxylic acid was extracted into chloroform. The chloroform solution was washed with water, dried ($MgSO_4$), and evaporated, and the residual gum was purified by HPLC ('Biosil', 20-44μ, $CH_2Cl_2$:MeOH:HOAc, 96.5:2.5:1.0). On treatment with ether, it gave crystals (0.8g), double m.p. 124° and 129-130°, $[\alpha]_D^{23}$ +22.56° (c = 1.02 in EtOH), of (5S)-5-(6-carboxyhexyl)-1-[(3R)-3-cyclohexyl-3-hydroxypropyl] hydantoin.

Treatment of diethyl (2S)-2-[(3S)-3-cyclohexyl-3-hydroxypropylamino] nonanedioate in the manner described above gave (5S)-5-(6-carboxyhexyl)-1-[(3S)-3-cyclohexyl-3-hydroxypropyl] hydantoin which, on exposure to ether at 0°, gave crystals, m.p. 72.5-73°, $[\alpha]_D^{18}$ - 17.0° (c = 1.03 in EtOH).

D. <u>Conversion of the epimers into (5R)-5-(6-carboxyhexyl)-1-[(3S)-3-cyclohexyl-3-hydroxypropyl)] hydantoin and (5R)-5-(6-carboxyhexyl)-1-[(3R)-3-cyclohexyl-3-hydroxypropyl] hydantoin</u>

(5S)-5-(6-Carboxyhexyl)-1-[(3S)-3-cyclohexyl-3-hydroxypropyl] hydantoin was taken up in 6 equivalents of N-aqueous sodium hydroxide and set aside at room temperature for 17 hours. The solution was acidified with hydrochloric acid, the product was taken into

BAD ORIGINAL

chloroform, and the chloroform solution was washed with water, dried ($MgSO_4$), and evaporated. The residual mixture of C-5 epimers was separated by HPLC yielding the starting compound and an equal quantity of (5R)-5-(6-carboxyhexyl)-1-[(3S)-3-cyclohexyl-3-hydroxypropyl]hydantoin which, on treatment with ether, gave crystals, double m.p. 124° and 128.5-130°, $[\alpha]_D^{19}$ -22.03° (c = 1.02 in EtOH).

Treatment of (5S)-5-(6-carboxyhexyl)-1-[(3R)-3-cyclohexyl-3-hydroxypropyl]hydantoin with 6 equivalents of N-aqueous sodium hydroxide at room temperature for 22 hours and work-up in the manner described above afforded (5R)-5-(6-carboxyhexyl)-1-[(3R)-3-cyclohexyl-3-hydroxypropyl]hydantoin which, on treatment with ether at 0°, gave crystals, m.p. 73-74.5°, $[\alpha]_D^{23}$ +16.93° (c = 1.0 in EtOH).

BAD ORIGINAL

In the following Examples, 'Compound 1' refers to (5S)-5-(6-carboxyhexyl)-1-[(3R)-3-hydroxy-3-cyclohexylpropyl]hydantoin.

EXAMPLE A

| Tablet | In one tablet |
|---|---|
| Compound 1 | 2.5 mg |
| Lactose B.P. | 87.5 mg |
| Starch B.P. | 10.0 mg |
| Povidone B.P.C. | 2.0 mg |
| Magnesium Stearate | 1.0 mg |

Mix together the Compound 1, lactose and starch. Granulate the powders using a solution of the povidone in Purified Water. Dry the granules, add the Magnesium Stearate and compress to produce tablets, 100 mg per tablet.

EXAMPLE B

| Capsule | In one capsule |
|---|---|
| Compound 1 | 5 mg |
| Lactose | 84 mg |
| Starch | 10 mg |
| Magnesium Stearate | 1 mg |

Mix the powders in a powder blender, fill into hard gelatine capsules, 100 mg per capsule.

EXAMPLE C

50 μg Single dose injection (freeze-dried)

| | |
|---|---|
| Compound 1 | 5.0 mg |
| Mannitol | 2.5 g |
| N/10 Sodium Hydroxide Solution | qs to pH 10.0 |
| Water for Injection | to ..... 100.0 ml |

BAD ORIGINAL

Suspend the Compound 1 in approximately 10 ml Water. Add sufficient Sodium Hydroxide Solution to produce pH 10 and stir to dissolve the Compound 1. Add and dissolve the Mannitol and dilute to volume with Water for Injection.

Sterilise the solution by passage through a membrane filter, 0.22 µm pore size and distribute aseptically into sterile vials, 1 ml per vial. Freeze dry the solutions and seal the containers under aseptic conditions with rubber closures. Each vial contains 50 µg Compound 1 as its freeze-dried Sodium salt.

EXAMPLE D - Inhibition of Platelet Aggregation

Aggregation of platelets in 0.5 ml of fresh human platelet rich plasma (PRP) was monitored in a Born aggregometer.

The compound to be tested was added to the PRP at the desired concentration, and the resulting mixture incubated at 37°C for 1 minute after which platelet aggregation was stimulated by the addition of adenosine diphosphate (ADP) in a concentration just causing maximal aggregation (2 to 5 µM).

The anti-aggregatory effect of the compound was assessed by measuring the percentage inhibition of platelet aggregation in the presence of the compound as compared with when it was completely absent. Thus the following relative potencies of the optical isomers of 5-(6-carboxyhexyl)-1-(3-hydroxy-3-cyclohexylpropyl) hydantoin were demonstrated with respect to $PGE_1$, on

BAD ORIGINAL

each of two experiments.

| Isomer | Inhibition of platelet aggregation x $PGE_1$ | |
|---|---|---|
| [(5S),(3R)] | 83 | 47 |
| [(5R),(3S)] | inactive ($IC_{50}$ > 2µg/ml) | |
| [(5S),(3S)] | 0.07 | 0.05 |
| [(5R),(3R)] | 0.05 | 0.04 |

1. A compound of formula (IA)

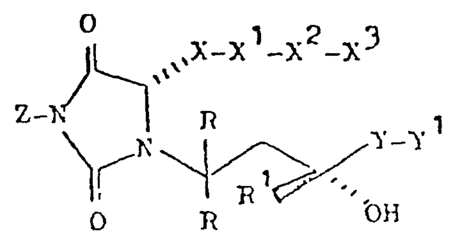

(IA)

wherein,

there are only two optically active centres;

Z is hydrogen or alkyl of 1 to 6 carbon atoms;

X is methylene, oxa (-O-) or thia (-S-);

$X^1$ is phenylene, -C≡C-, <u>cis</u> or <u>trans</u> -CH=CH- or $-CH_2-CH_2-$;

$X^2$ is a covalent bond or a straight or branched alkylene chain having 1 to 6 carbon atoms;

$X^1$ or $X^2$ optionally having one of any methylene groups replaced by oxa or thia provided that at least one carbon atom separates the oxa or thia from any 5-tetrazolyl or carbonyl or -C≡C- or <u>cis</u> or <u>trans</u> -CH=CH- group, further provided that $-X-X^1-X^2-$ contains a maximum of one of either an oxa or thia;

$X^3$ is selected from 5-tetrazolyl, carboxyl, carbamoyl, alkoxycarbonyl and hydroxymethyl;

each R is the same and is selected from hydrogen and methyl;

$R^1$ is hydrogen or alkyl;

Y is a covalent bond or straight or symmetrically branched alkylene having 1 to 7 carbon atoms optionally substituted at the carbon adjacent $-C<^{R^1}_{OH}$ by two groups

each of which may be alkyl or a cyclic radical provided no optical centre is introduced;

$Y^1$ is hydrogen, alkoxy of 1 to 7 carbon atoms, a cyclic radical, phenyl, benzyl, phenoxy or benzyloxy, wherein each of phenyl, benzyl, phenoxy and benzyloxy may be substituted in the benzene ring by one or more groups selected from hydroxy, halo, nitro, amino, acylamino, alkenyl, alkoxy, phenyl, benzyloxy and alkyl which may itself be substituted by one or more halo groups; or Y and $Y^1$ together form an alkyl group of 1 to 7 carbon atoms having at least one hydrogen replaced by fluoro;

and salts thereof

in substantially pure form as hereinbefore defined.

2. (5<u>S</u>)-5-(6-Carboxyhexyl)-1-/(3<u>R</u>)-3-hydroxy-3-cyclohexylpropyl7 hydantoin or an ester or a salt thereof, in substantially pure form as hereinbefore defined.

3. A method of preparing a compound of formula ( IA ) as defined in claim 1 or claim 2 comprising cyclization under acidic conditions or by heating of a compound of formula (IXA):

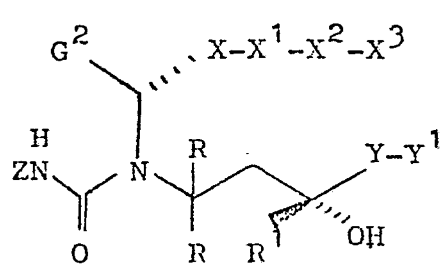

(IXA)

wherein,

Z, X to $X^3$, R, $R^1$, Y and $Y^1$ are as defined in formula ( 1A ) and

BAD ORIGINAL

$G^2$ is G or $G^1$ wherein G is alkoxycarbonyl and $G^1$ is carboxyl.

4. A method of preparing a compound of formula ( IA ) as defined in claim 1 or claim 2 comprising

( a ) selective hydrolysis, in the presence of a carboxylic acid, of a compound of formula ( II )

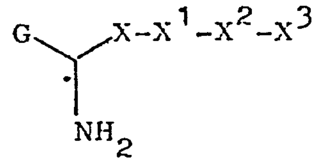

(II)

wherein X to $X^2$ are as defined in formula ( IA ); and G and $X^3$ are the same or different and each is alkoxycarbonyl

to produce a compound of formula ( III )

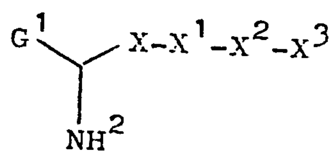

(III)

wherein X to $X^3$ are as defined in formula ( II ): and $G^1$ is carboxyl;

( b ) acylation of the compound of formula ( III ) to produce a compound of formula ( IV )

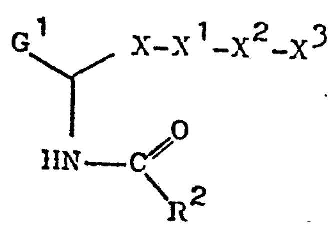

(IV)

BAD ORIGINAL

wherein $G^1$ and X to $X^3$ are as defined in formula ( III ); and $R^2$ is $-(CH_2)_nQ$ wherein n is 1, 2 or 3 and Q is hydrogen or halo;

( c ) selective deacylation of the compound of formula ( IV ) to produce a compound of formula ( IIIA )

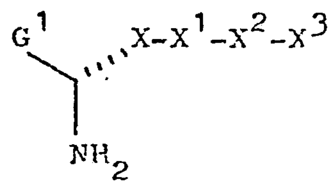

(IIIA)

wherein $G^1$ and X to $X^3$ are as defined in formula ( IV ); and

( d ) conversion of the compound of formula ( IIIA ) so produced to the compound of formula ( IA ).

5. A composition comprising a compound of formula ( IA ) as defined in claim 1 or claim 2 in association with a pharmaceutically acceptable carrier.

6. A compound of formula ( IA ) as defined in claim 1 or claim 2 for use in the inhibition of platelet aggregation.

7. A compound of formula ( IA ) as defined in claim 1 or claim 2 for use in inducing bronchodilation in a mammal.

8. A compound of formula ( IA ) as defined in claim 1 or claim 2 for use in inducing vasodilation in a mammal.

BAD ORIGINAL

9. A compound of formula ( IXA )

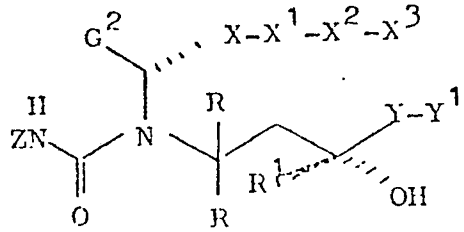

(IXA)

wherein,

there are only two optically active centres;

Z is hydrogen or alkyl of 1 to 6 carbon atoms;

$G^2$ is G or $G^1$ wherein G is alkoxycarbonyl and $G^1$ is carboxyl

X is methylene, oxa (-O-) or thia (-S-);

$X^1$ is phenylene, -C≡C-, cis or trans -CH=CH- or $-CH_2-CH_2-$;

$X^2$ is a covalent bond or a straight or branched alkylene chain having 1 to 6 carbon atoms;

$X^1$ or $X^2$ optionally having one of any methylene groups replaced by oxa or thia provided that at least one carbon atom separates the oxa or thia from any 5-tetrazolyl or carbonyl or -C≡C- or cis or trans -CH=CH- group, further provided that $-X-X^1-X^2-$ contains a maximum of one of either an oxa or thia;

$X^3$ is selected from 5-tetrazolyl, carboxyl, carbamoyl, alkoxycarbonyl and hydroxymethyl;

each R is the same and is selected from hydrogen and methyl;

$R^1$ is hydrogen or alkyl;

Y is a covalent bond or straight or symmetrically branched alkylene having 1 to 7 carbon atoms optionally

BAD ORIGINAL

substituted at the carbon adjacent 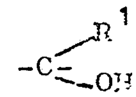 by two groups each of which may be alkyl or a cyclic radical provided no optical centre is introduced;

$Y^1$ is hydrogen, alkoxy of 1 to 7 carbon atoms, a cyclic radical, phenyl, benzyl, phenoxy, or benzyloxy, wherein each of phenyl, benzyl, phenoxy and benzyloxy may be substituted in the benzene ring by one or more groups selected from hydroxy, halo, nitro, amino, acylamino, alkenyl, alkoxy, phenyl, benzyloxy and alkyl which may itself be substituted by one or more halo groups; or Y and $Y^1$ together form an alkyl group of 1 to 7 carbon atoms having at least one hydrogen replaced by fluoro.

10. A compound of formula

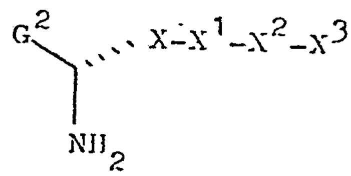

wherein

X is methylene, oxa (-O-) or thia (-S-);

$X^1$ is phenylene, -C≡C-, <u>cis</u> or <u>trans</u> -CH=CH- or $-CH_2-CH_2-$;

$X^2$ is a covalent bond or a straight or branched

BAD ORIGINAL

alkylene chain having 1 to 6 carbon atoms;

$X^1$ or $X^2$ optionally having one of any methylene groups replaced by oxa or thia provided that at least one carbon atom separates the oxa or thia from any 5-tetrazolyl or carbonyl or -C≡C- or cis or trans -CH=CH- group, further provided that $-X-X^1-X^2-$ contains a maximum of one of either an oxa or thia;

$X^3$ is selected from 5-tetrazolyl, carboxyl, carbamoyl, alkoxycarbonyl and hydroxymethyl;

and $G^2$ is G or $G^1$ wherein G is alkoxycarbonyl and $G^1$ is carboxyl.

BAD ORIGINAL

0019223

European Patent Office

# EUROPEAN SEARCH REPORT

Application number
EP 80102536.2

| DOCUMENTS CONSIDERED TO BE RELEVANT | | | CLASSIFICATION OF THE APPLICATION (Int. Cl. 3) |
|---|---|---|---|
| Category | Citation of document with indication, where appropriate of relevant passages | Relevant to claim | |
| | DE - A1 - 2 724 948 (WELLCOME)<br>+ Pages 14-22, 32-41, 73-76 +<br>-- | 1,3,5-8 | C 07 D 233/78<br>A 61 K 31/415<br>C 07 C 127/15<br>C 07 C 101/20 |
| | DE - A1 - 2 755 771 (BEECHAM)<br>+ Pages 13-33 +<br>-- | 1,3,5-8 | |
| P | DE - A1 - 2 922 070 (WELLCOME) (06-12-1979)<br>+ Pages 10-42 +<br>& FR-A2-2 427 331 (28-12-1979)<br>& SE-A -7 904 700 (01-12-1979)<br>-- | 1,3,5-8 | TECHNICAL FIELDS SEARCHED (Int.Cl. 3) |
| P | EP - A2 - 0 002 258 (WELLCOME) (13-06-1979)<br>+ Pages 1-8, 14-33 +<br>-- | 1,3-5-8 | C 07 D 233/00<br>A 61 K 31/00<br>C 07 C 127/00<br>C 07 C 101/00 |
| P | EP - A1 - 0 003 410 (BEECHAM) (08-08-1979)<br>+ Pages 1-17 +<br>---- | 1,3,5-8 | |

CATEGORY OF CITED DOCUMENTS
X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| VIENNA | 30-06-1980 | PETROUSEK |

EPO Form 1503.1 06.78